(19) Europäisches Patentamt
European Patent Office
Office européen des brevets

(11) **EP 4 628 119 A1**

(12) **EUROPÄISCHE PATENTANMELDUNG**

(43) Veröffentlichungstag:
**08.10.2025 Patentblatt 2025/41**

(21) Anmeldenummer: **25165308.5**

(22) Anmeldetag: **21.03.2025**

(51) Internationale Patentklassifikation (IPC):
**A61M 1/16** (2006.01)

(52) Gemeinsame Patentklassifikation (CPC):
**A61M 1/1658; A61M 1/166;** A61M 1/1664;
A61M 2205/3331; A61M 2205/3368;
A61M 2205/3389; A61M 2205/70

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(30) Priorität: **28.03.2024 DE 102024109052**

(71) Anmelder: **B. Braun Avitum AG
34212 Melsungen (DE)**

(72) Erfinder:
• **JANIK, Waldemar
34212 Melsungen (DE)**
• **LINDNER, Joshua
34212 Melsungen (DE)**

(74) Vertreter: **Maiwald GmbH
Elisenhof
Elisenstraße 3
80335 München (DE)**

(54) **COMPUTER-IMPLEMENTIERTES VERFAHREN FÜR EIN DIALYSESYSTEM**

(57) Die Erfindung betrifft ein computer-implementiertes Verfahren für ein Dialysesystem zum Erzeugen von Steuerdaten zum druckbasierten Steuern des Dialysesystems, wobei das Verfahren umfasst: Überwachen des Drucks in einem Entgasungsabschnitt eines Fluidsystem des Dialysesystems, Erzeugen von Steuerdaten, wenn erkannt wird, dass der Druck mindestens ein Kriterium erfüllt, wobei das Erzeugen der Steuerdaten ein Erzeugen eines Heizvorrichtungssteuersignals für eine Heizvorrichtung des Dialysesystems umfasst.

Fig. 2

EP 4 628 119 A1

**Beschreibung**

TECHNISCHES GEBIET

Die Erfindung betrifft ein computer-implementiertes Verfahren für ein Dialysesystem, ein System zur Datenverarbeitung, ein Dialysesystem umfassend das System zur Datenverarbeitung, ein Computerprogrammprodukt und ein computerlesbares Medium.

HINTERGRUND DER ERFINDUNG

In Dialysesystemen ist ein Ansteuern der verschiedenen Komponenten stets eine Herausforderung. Insbesondere das Steuern von Heizvorrichtungen, aber auch von Ventilen, ist dabei besonders wichtig.

Aktuelle Systeme setzen beispielsweise beim Steuern des Heizvorgangs auf das Abfragen eines Füllstandes in einer Kammer. Häufig kommt ein mechanischer Niveausensor mit einem Schwimmer zum Einsatz. Beispielsweise kann die Steuerung derart erfolgen, dass erst dann, wenn mittels des Niveausensors erkannt wurde, dass in der Kammer ein entsprechender Füllstand erreicht wurde, die Heizvorrichtung eingeschaltet wird. Als weiteres Beispiel kann die Steuerung derart erfolgen, dass die Heizvorrichtung ausgeschaltet wird, sobald der Füllstand unterschritten wurde. Die Steuerung von Ventilen, besonders einem Einlassventil, ist neben der Heizungssteuerung ebenfalls relevant und häufig an Messwerte des Niveausensors gekoppelt, weil ein Ventilzustand das Zu- und/oder Ablaufen der Flüssigkeit und somit den Füllstand mitbestimmt.

Die Nutzung von mechanischen Niveausensoren hat allerdings den Nachteil, dass diese teilweise nicht zuverlässig sind, insbesondere fehleranfällig in der Hinsicht, dass der Schwimmer klemmt, wodurch ein falscher Füllstand registriert werden würde. Es besteht also Bedarf einer zuverlässigeren Steuerung der Heizvorrichtung und ggf. von Ventilen.

Ein Betreiben der Heizvorrichtung bei zu wenig Flüssigkeit, kann Schäden im System hervorrufen und/oder zu Fehlfunktionen führen, die teilweise auch sicherheitsrelevant sind. Es besteht Bedarf, solche Schäden und Fehlfunktionen zuverlässiger zu vermeiden.

Der Erfindung liegt die Aufgabe zugrunde, ein computer-implementiertes Verfahren für ein Dialysesystem bereitzustellen, das zumindest manche der oben ausgeführten Probleme adressiert, insbesondere eine zuverlässige Steuerung ermöglicht und/oder Schäden und/oder Fehlfunktionen zuverlässig vermeidet.

ZUSAMMENFASSUNG DER ERFINDUNG

Die Erfindung stellt ein computer-implementiertes Verfahren für ein Dialysesystem, ein System zur Datenverarbeitung, ein Dialysesystem umfassend das System zur Datenverarbeitung, ein Computerprogrammprodukt und ein computerlesbares Medium gemäß den unabhängigen Ansprüchen bereit. Ausführungsformen sind unter anderem in den abhängigen Ansprüchen erläutert.

Die Erfindung betrifft ein computer-implementiertes Verfahren für ein Dialysesystem zum Erzeugen von Steuerdaten zum druckbasierten Steuern des Dialysesystems, wobei das Verfahren umfasst: Überwachen des Drucks in einem Entgasungsabschnitt eines Fluidsystem des Dialysesystems, Erzeugen von Steuerdaten, wenn erkannt wird, dass der Druck mindestens ein Kriterium erfüllt, wobei das Erzeugen der Steuerdaten ein Erzeugen eines Heizvorrichtungssteuersignals für eine Heizvorrichtung des Dialysesystems umfasst. In dem Entgasungsabschnitt kann Flüssigkeit, beispielsweise Wasser, temperiert werden.

Mit anderen Worten wird ein Verfahren bereitgestellt, das Steuerdaten erzeugt, mittels derer die Heizvorrichtung des Dialysesystems druckabhängig gesteuert werden kann, insbesondere beispielsweise druckabhängig gestartet und/oder gestoppt. Das Steuern der Heizvorrichtung wird dabei mittels des Heizvorrichtungssteuersignals erreicht. Das Heizvorrichtungssteuersignal wird abhängig vom Druck erzeugt. Die Steuerdaten, insbesondere das Heizvorrichtungssteuersignal, werden erzeugt, wenn erkannt wird, dass der Druck, der im Entgasungsabschnitt überwacht wird, ein Kriterium erfüllt, beispielsweise zu schnell und/oder zu weit absinkt und/oder ansteigt.

Der Druck im Entgasungsabschnitt ist ein Indikator dafür, ob im Fluidsystem, insbesondere in einem vorgeschalteten Behälter, aus dem Flüssigkeit in den Entgasungsabschnitt eintritt, zumindest eine vorgegebene Mindestmenge an Flüssigkeit vorhanden ist. Somit kann also indirekt aus Druckmesswerten auf den Füllstand geschlossen werden, was eine zuverlässigere Steuerung ermöglicht, da die Druckmessungen alternativ oder zusätzlich zu dem möglicherweise unzuverlässigen Niveausensor verwendet werden können.

Das Dialysesystem kann derart ausgebildet sein, dass im Regelbetrieb die Flüssigkeit durch den Entgasungsabschnitt strömt, bevor sie in die Heizvorrichtung zum Heizen eintritt. Wenn also im Entgasungsabschnitt eine Druckveränderung stattfindet, die darauf hindeutet, dass zu wenig Flüssigkeit im Fluidsystem vorhanden ist, dann deutet dies insbesondere auch darauf hin, dass auch in einem Heizbehälter, in dem die Flüssigkeit durch die Heizvorrichtung erwärmt wird, mit erhöhter Wahrscheinlichkeit zu wenig Flüssigkeit vorhanden ist. Dies kann aus den oben erläuterten Gründen zuverlässiger erkannt werden als bisher. So kann auch das Risiko reduziert werden, dass die Heizvorrichtung bei zu niedriger Flüssigkeitsmenge heizt und somit das Risiko von Schäden und Fehlfunktionen reduzieren.

Ein Koppeln des Betriebs der Heizvorrichtung (und optional der Ventile) an den Druck im Entgasungsabschnitt erlaubt also, zumindest die oben genannten Probleme zu adressieren, insbesondere eine zuverlässige Steuerung zu ermöglichen und/oder Schäden un-

Hier sei angemerkt, dass sich in der vorliegenden Offenbarung die Merkmale des Verfahrens, soweit nicht explizit anders angegeben, insbesondere auf den bestimmungsgemäßen Betrieb des bestimmungsgemäß ausgebildeten Dialysesystems richten.

Das Dialysesystem kann zumindest zum Sammeln, Entgasen und Erwärmen von Flüssigkeit im Rahmen einer extrakorporalen Blutbehandlung ausgebildet sein. Das Dialysesystem kann zudem zur Zudosierung von Konzentraten zur Herstellung von Dialysierflüssigkeit und/oder zur Bilanzierung samt Ultrafiltration ausgebildet sein. Das Dialysesystem kann eine Blutpumpe, einen Dialysator, einen Drucksensor und/oder ein Schlauchsystem umfassen. Das Dialysesystem kann auch einen oder mehrere Aktoren und/oder ein oder mehrere (weiter) Sensoren zur Durchführung einer Dialysebehandlung umfassen.

Ein Erzeugen von Steuerdaten zum Steuern des Dialysesystems kann gemäß der vorliegenden Offenbarung zumindest das Erzeugen eines Steuersignals für eine Komponente des Dialysesystems umfassen, beispielsweise des Heizvorrichtungssteuersignals. Die Steuerdaten können für eine oder mehrere Komponenten des Dialysesystems jeweils ein oder mehrere Steuersignale umfassen.

Im Folgenden wird für die jeweiligen Steuersignale für verschiedene Komponenten (beispielsweise Heizvorrichtungssteuersignal und Ventilsteuersignal) kollektiv der Begriff "Steuersignal" verwendet.

In der vorliegenden Offenbarung wird insbesondere davon ausgegangen, dass das Steuersignal unmittelbar, also ohne Zeitverzug, zum Steuern der Komponente mittels des Steuersignals verwendet wird.

Das Erfüllen des Kriteriums kann ein sofortiges Erzeugen der Steuerdaten, insbesondere des Steuersignals, auslösen. Dies wird auch als Auslösen ohne Verzögerung bezeichnet. Optional können die sofort erzeugten Steuerdaten, insbesondere das Steuersignal, eine vorgegebene Verzögerung umfassen, nach der die Komponente eine durch das Steuersignal vorgegebene Aktion durchführen soll, beispielsweise den Komponentenbetrieb starten, den Komponentenbetrieb stoppen, öffnen (wenn die Komponente ein Ventil ist), und/oder schließen (wenn die Komponente ein Ventil ist). Für die Steuersignale für verschiedene Komponenten kann eine etwaige durch das jeweilige Steuersignal vorgegebene Verzögerung unterschiedlich sein.

Der Drucksensor bzw. die Drucksensoren können in einem oder mehreren Bereichen des Entgasungsabschnitts angeordnet sein.

Der Entgasungsabschnitt kann eine Entgasungskammer, eine Pumpe und eine Drossel umfassen. Der Druck kann beispielsweise in Flussrichtung zwischen der Drossel und der Pumpe gemessen werden. Die Entgasungskammer kann in Flussrichtung zwischen dem Bereich der Druckmessung und der Pumpe angeordnet sein.

Dem Entgasungsabschnitt kann in Flussrichtung ein Flüssigkeitsbehälter vorgelagert sein, aus dem im Betrieb Flüssigkeit dem Entgasungsabschnitt zugeführt wird. Wenn dieser ausreichend gefüllt ist und die Pumpe läuft, wird in dem Entgasungsabschnitt ein Druckwert eingenommen, der niedriger ist als der Druckwert bei leerem Flüssigkeitsbehälter. Eine Druckveränderung deutet also auf eine Füllstandsänderung im Behälter hin.

Das Überwachen des Drucks kann ein, beispielsweise regelmäßiges oder kontinuierliches, Erfassen von Messwerten eines oder mehrere Drucksensoren umfassen. Das Überwachen des Drucks kann umfassen, dass mindestens ein Drucksensor im Entgasungsabschnitt angeordnet ist und in, z.B. regelmäßigen, Abständen oder kontinuierlich den Druck misst und Messwerte bereitstellt. Es kann sich um eine in diesem Bereich übliche Entgasungskammer handeln.

Das Verfahren kann umfassen, dass anhand der durch das Überwachen des Drucks erhaltenen Werte geprüft wird, ob der Druck das (mindestens eine) Kriterium erfüllt. Das Kriterium kann sich auf die erhaltenen Werte selbst beziehen oder auf aus den erhaltenen Werten abgeleitete Daten, beispielsweise durch Mittelung, Ermitteln der Steigung, Ermitteln der Standardabweichung, Integration, oder dergleichen. Abgeleitete Daten können beispielsweise Daten umfassen, die einen zeitlichen Verlauf der erhaltenen Werte repräsentieren.

Das (mindestens eine) Kriterium kann zur Prüfung aus einem Speicher abgerufen werden. Das Kriterium kann ein theoretisch bestimmtes Kriterium sein, beispielsweise durch Modellierung des Dialysesystems, oder ein empirisch bestimmtes Kriterium oder ein semiempirisch bestimmtes Kriterium sein. Das Kriterium kann sich beispielsweise von der Dimensionierung des Systems und den Eigenschaften der verbauten Komponenten ableiten. Alternativ kann das Kriterium unabhängig von den Dimensionierungen und Eigenschaften gewählt sein, also derart, dass es für alle erwartbaren Dimensionierungen und Eigenschaften geeignet ist.

Beispiele für das anspruchsgemäße Kriterium werden unten erläutert.

Die Heizvorrichtung des Dialysesystems kann insbesondere eine elektrisch betriebene Heizung umfassen. Die Heizvorrichtung kann derart ausgebildet und angeordnet sein, dass sie im bestimmungsgemäßen Betrieb Flüssigkeit, insbesondere Flüssigkeit, die im Heizbehälter steht oder durch den Heizbehälter strömt, zu heizen. Beispielsweise kann die Heizvorrichtung einen Heizstab umfassen, der in den Heizbehälter ragt, oder ein Heizelement, beispielsweise Heizrohr, umfassen, das beispielsweise integral mit der Behälterwand ausgebildet oder an der der Behälterwand angeordnet sein kann. Alternative Ausgestaltungen sind ebenfalls denkbar.

Gemäß der vorliegenden Offenbarung kann das mindestens eine Kriterium ein erstes Kriterium, das sich auf einen Druckwert bezieht, umfassen.

Ein solches Kriterium kann auch als statisches Kriterium betrachtet werden, das sich auf einen Zustand des Drucks zu einem Zeitpunkt oder in einem Zeitintervall mit vorgegebener Länge bezieht. Die Verwendung eines statischen Kriteriums ermöglicht eine einfache, effiziente und zuverlässige Prüfung, ob das Kriterium erfüllt ist. Beispielsweise kann das Kriterium sein, dass der Druckwert in einem Zeitintervall einem Solldruckwert entspricht oder in einem Soll-Intervall liegt oder über oder unter einem Sollwert liegt. Dies ist unten im Detail beschrieben.

Alternativ oder zusätzlich kann das mindestens eine Kriterium ein zweites Kriterium, das sich auf eine Druckänderung, insbesondere einen Druckanstieg und/oder einen Druckabfall, bezieht, umfassen.

Ein solches Kriterium kann auch als dynamische Kriterium betrachtet werden, das sich auf die Änderung, beispielsweise Abfall oder Anstieg, des Druckwerts bezieht, insbesondere über ein Zeitintervall mit vorgegebener Länge. Ein dynamisches Kriterium ermöglicht eine Prüfung, die weniger anfällig für einzelne Messfehler ist. Je nach Kriterium ist es sogar möglich, korrekte Ergebnisse zu erhalten, selbst wenn beispielsweise der absolute gemessene Druck nicht korrekt ist, da die Änderung unabhängig vom absoluten Wert ist.

Ein Druckwert kann gemäß der vorliegenden Offenbarung insbesondere ein Messwert eines oder mehrerer Drucksensoren sein oder ein aus einem oder mehreren Messwerten eines oder mehrere Drucksensoren ermittelter Druckwert sein. Beispielsweise kann der Druckwert ein aus mehreren Messwerten eines Drucksensors statistisch ermittelter Druckwert sein, beispielsweise ein Mittelwert über ein vorgegebenes Zeitintervall.

Gemäß der vorliegenden Offenbarung kann das mindestens eine Kriterium, insbesondere das erste Kriterium bzw. das statische Kriterium, ein Kriterium umfassen, dass ein tatsächlicher Druckwert einem Solldruckwert entspricht, optional über ein Zeitintervall mit vorgegebener Länge. Alternativ oder zusätzlich kann das mindestens eine Kriterium, insbesondere das erste Kriterium bzw. statische Kriterium, ein Kriterium umfassen, dass der Druckwert einem Solldruckwert entspricht, in einem Soll-Intervall liegt, und/oder größer als der Solldruckwert oder kleiner als der Solldruckwert ist, optional jeweils über ein Zeitintervall mit vorgegebener Länge.

Alternativ oder zusätzlich kann, gemäß der vorliegenden Offenbarung, das mindestens eine Kriterium, insbesondere das zweite Kriterium oder dynamische Kriterium, ein Kriterium umfassen, dass ein tatsächlicher Druckwert unter einen vorgegebenen Druckwert fällt oder dass ein tatsächlicher Druckwert über einen vorgegebenen Druckwert steigt oder dass ein tatsächlicher Druckwert sich mindestens um einen vorgegebenen Betrag ändert, insbesondere mindestens um den vorgegebenen Betrag steigt oder fällt, insbesondere innerhalb eines Zeitintervalls vorgegebener Länge. Alternativ oder zusätzlich kann das mindestens eine Kriterium, insbesondere das zweite Kriterium bzw. dynamische Kriterium, ein Kriterium umfassen, dass sich der Druckwert schneller als eine vorgegebene Solländerungsrate ändert, insbesondere innerhalb eines Intervalls vorgegebener Länge und/oder mindestens um einen vorgegebenen Mindestbetrag.

Alternativ oder zusätzlich kann, gemäß der vorliegenden Offenbarung, das mindestens eine Kriterium, insbesondere das zweite Kriterium bzw. dynamische Kriterium, ein Kriterium umfassen, dass eine gleitende Standardabweichung des Drucks über einen vorgegebenen Wert steigt. Dies Standardabweichung bezieht sich dabei auf mehrere über einen Zeitraum nacheinander gemessene Druckwerte.

Wenn ein Kriterium verwendet wird, das beispielsweise auf den Betrag der Änderung und/oder die Änderungsrate der Änderung des Druckwertes gerichtet ist, ermöglicht das vorteilhaft, unabhängig von einer absoluten Druckmessung zuverlässige Ergebnisse zu erhalten.

Wenn sich ein Kriterium auf die gleitende Standardabweichung bezieht, so ermöglicht dies, Ausreißer oder Schwankungen bei Messwerten nicht als Auslöser für die Erzeugung des Steuersignals zu verwenden, sondern beispielsweise echte Druckänderungen zuverlässig zu erkennen und nur basierend auf solchen echten Druckänderungen Steuerdaten zu erzeugen. Die Verwendung der Standardabweichung ist insbesondere deshalb vorteilhaft, weil das Ansaugen von Luftblasen den Istwert deutlich schwanken lassen kann.

Gemäß der vorliegenden Offenbarung kann das mindestens eine Kriterium ein Startkriterium für das Starten der Heizvorrichtung umfassen, wobei, wenn der Druck ein Startkriterium erfüllt, ein Startsignal als Heizvorrichtungssteuersignal erzeugt wird.

Das heißt, wenn erkannt wird, dass das Startkriterium erfüllt ist, kann unmittelbar oder mit einer vorgegebenen Verzögerung (beispielsweise gemäß dem Steuersignal, hier gemäß dem Startsignal) das Starten der Heizvorrichtung ausgelöst werden. Das Starten der Heizvorrichtung ist hier insbesondere als Starten eines Heizvorgangs der Heizvorrichtung zu betrachten.

Wie oben allgemein für die Steuersignale erläutert, kann das Startsignal sofort erzeugt werden. Das Startsignal kann derart ausgebildet sein, dass es die Heizvorrichtung veranlasst, sofort oder nach einer durch das Startsignal vorgegebenen Verzögerung zu starten, insbesondere den Heizvorgang zu starten.

Das Startkriterium kann insbesondere derart gewählt sein, dass es sich auf einen Druckwert und/oder eine Druckänderung bezieht, also beispielsweise das oben erläuterte erste und/oder das oben erläuterte zweite Kriterium umfassen. Insbesondere kann das Startkriterium derart gewählt sein, dass der Druckwert und/oder die Druckänderung, beispielsweise generell oder für das vorliegende System, anzeigt, dass Flüssigkeit im dem Entgasungsabschnitt in Flussrichtung vorgelagerten Behälter vorhanden ist.

Alternativ oder zusätzlich zu einem Startkriterium kann, gemäß der vorliegenden Offenbarung, das mindestens eine Kriterium ein Stoppkriterium für das Stoppen der Heizvorrichtung umfassen, wobei, wenn der Druck ein Stoppkriterium erfüllt, ein Stoppsignal als Heizvorrichtungssteuersignal erzeugt wird.

Das heißt, wenn erkannt wird, dass das Stoppkriterium erfüllt ist, kann unmittelbar oder mit einer vorgegebenen Verzögerung (beispielsweise gemäß dem Steuersignal, hier gemäß dem Stoppsignal) das Stoppen der Heizvorrichtung ausgelöst werden. Das Stoppen der Heizvorrichtung ist hier insbesondere als Stoppen eines Heizvorgangs der Heizvorrichtung zu betrachten.

Wie oben allgemein für die Steuersignale erläutert, kann das Stoppsignal sofort erzeugt werden. Das Stoppsignal kann derart ausgebildet sein, dass es die Heizvorrichtung veranlasst, sofort oder nach einer durch das Stoppsignal vorgegebenen Verzögerung zu stoppen, insbesondere den Heizvorgang zu stoppen.

Das Stoppkriterium kann insbesondere derart gewählt sein, dass es sich auf einen Druckwert und/oder eine Druckänderung bezieht, also beispielsweise das oben erläuterte erste und/oder das oben erläuterte zweite Kriterium umfassen. Insbesondere kann das Stoppkriterium derart gewählt sein, dass der Druckwert und/oder die Druckänderung, beispielsweise generell oder für das vorliegende System, anzeigt, dass keine oder zu wenig Flüssigkeit im dem Entgasungsabschnitt in Flussrichtung vorgelagerten Behälter vorhanden ist.

Alternativ oder zusätzlich zu einem Startkriterium und/oder Stoppkriterium kann, gemäß der vorliegenden Offenbarung, das mindestens eine Kriterium ein Anpassungskriterium für das Anpassen des Betriebs der Heizvorrichtung umfassen, wobei, wenn der Druck ein Anpassungskriterium erfüllt, ein Anpassungssignal als Heizvorrichtungssteuersignal erzeugt wird.

Das heißt, wenn erkannt wird, dass das Anpassungskriterium erfüllt ist, kann unmittelbar oder mit einer vorgegebenen Verzögerung (beispielsweise gemäß dem Steuersignal, hier gemäß dem Anpassungssignal) der Betrieb der Heizvorrichtung angepasst werden. Das Anpassen kann umfassen, das ein oder mehrere Betriebsparameter der im Betrieb befindlichen Heizvorrichtung geändert werden. Beispielsweise kann das Anpassen umfassen, eine Heizleistung und/oder eine Solltemperatur als Betriebsparameter der Heizvorrichtung zu ändern.

Wie oben allgemein für die Steuersignale erläutert, kann das Anpassungssignal sofort erzeugt werden. Das Anpassungssignal kann derart ausgebildet sein, dass es die Heizvorrichtung veranlasst, sofort oder nach einer durch das Anpassungssignal vorgegebenen Verzögerung das Anpassen durchzuführen, insbesondere den/die Betriebsparameter zu ändern.

Das Anpassungssignal kann insbesondere derart gewählt sein, dass es sich auf einen Druckwert und/oder eine Druckänderung bezieht, also beispielsweise das oben erläuterte erste und/oder das oben erläuterte zweite Kriterium umfassen. Insbesondere kann das Anpassungssignal derart gewählt sein, dass der Druckwert und/oder die Druckänderung, beispielsweise generell oder für das vorliegende System, anzeigt, dass sich die Flüssigkeitsmenge im dem Entgasungsabschnitt in Flussrichtung vorgelagerten Behälter verändert hat, beispielsweise um mehr als einen Schwellwert geändert.

Gemäß der vorliegenden Offenbarung kann das mindestens eine Kriterium das Startkriterium umfassen und das Startkriterium kann umfassen, dass ein tatsächlicher Druckwert unter einen vorgegebenen Druckwert fällt oder dass ein tatsächlicher Druckwert mindestens um einen vorgegebenen Betrag fällt oder dass eine gleitende Standardabweichung des Drucks über einen vorgegebenen Wert steigt. Die zeitliche Standardabweichung kann sich dabei auf mehrere über einen Zeitraum hinweg nacheinander erfasste Druckwerte beziehen.

Insbesondere ist eine Druckänderung besonders aussagekräftig, da eine Änderung darauf hindeutet, dass der vorangegangene Zustand, also Vorhandensein oder Fehlen von Flüssigkeit, sich signifikant ändert bzw. geändert hat.

Gemäß der vorliegenden Offenbarung kann das Erzeugen der Steuerdaten ein Erzeugen eines Ventilsteuersignals für ein Einlassventil des Dialysesystems umfassen, welches die Flüssigkeitszufuhr in ein Fluidsystem des Dialysesystems steuert.

Bei bestimmungsgemäßer Verwendung wird durch das Öffnen des Einlassventil Zufluss von Flüssigkeit in das Fluidsystem, insbesondere in den/einen dem Entgasungsabschnitt in Flussrichtung vorgelagerten Behälter der Teil des Fluidsystems ist, gesteuert. Somit kann das Ventilsteuersignal dazu verwendet werden, durch entsprechendes Ansteuern des Einlassventils, eine Flüssigkeitszufuhr zu starten oder zu stoppen. Wie oben schon erläutert ist der Druck, insbesondere der Druckwert und/oder eine Druckänderung, ein Indikator für das Vorhandensein oder Fehlen von Flüssigkeit. Das Erzeugen des Ventilsteuersignals an den Druck zu koppeln ermöglicht also, Flüssigkeit zuzuführen, wenn aufgrund des Drucks von einem Fehlen von Flüssigkeit auszugehen ist, und/oder die Zufuhr von Flüssigkeit zu unterbrechen, wenn aufgrund des Drucks von Vorhandensein einer ausreichenden Menge von Flüssigkeit auszugehen ist. Entsprechende Kriterien werden unten erläutert.

Wenn das Erzeugen der Steuerdaten ein Erzeugen eines Ventilsteuersignals für ein Einlassventil des Dialysesystems umfasst, welches die Flüssigkeitszufuhr in ein Fluidsystem des Dialysesystems steuert, kann das mindestens eine Kriterium ein Öffenkriterium umfassen, wobei, wenn der Druck das Öffenkriterium erfüllt, ein Signal zum Öffnen des Einlassventils erzeugt wird. Alternativ oder zusätzlich kann das mindestens eine Kriterium ein Schließkriterium umfassen, wobei, wenn der Druck das Schließkriterium erfüllt, ein Signal zum Schließen des Einlassventils erzeugt wird.

Das heißt, wenn erkannt wird, dass das Öffenkriterium erfüllt ist, kann unmittelbar oder mit einer vorgegebenen Verzögerung das Öffnen des Einlassventils ausgelöst werden, und/oder wenn das Schließkriterium erfüllt ist, kann unmittelbar oder mit einer vorgegebenen Verzögerung das Schließen des Einlassventils ausgelöst werden. Wie oben allgemein für die Steuersignale erläutert, kann das Ventilsteuersignal sofort erzeugt werden. Das Ventilsteuersignal kann derart ausgebildet sein, dass es das Einlassventil veranlasst, sofort oder nach einer durch das Ventilsteuersignal vorgegebenen Verzögerung zu öffnen bzw. zu schließen.

Das Öffenkriterium und/oder das Schließkriterium kann jeweils insbesondere derart gewählt sein, dass es sich auf einen Druckwert oder eine Druckänderung bezieht, also beispielsweise das oben erläuterte erste oder das oben erläuterte zweite Kriterium umfassen. Insbesondere kann das Öffenkriterium derart gewählt sein, dass der Druckwert oder die Druckänderung, beispielsweise generell oder für das vorliegende System, anzeigt, dass keine oder zu wenig Flüssigkeit im dem Entgasungsabschnitt in Flussrichtung vorgelagerten Behälter vorhanden ist. Alternativ oder zusätzlich kann das Schließkriterium derart gewählt sein, dass der Druckwert oder die Druckänderung, beispielsweise generell oder für das vorliegende System, anzeigt, dass ausreichend Flüssigkeit im dem Entgasungsabschnitt in Flussrichtung vorgelagerten Behälter vorhanden ist.

Das Verfahren der vorliegenden Offenbarung kann ein Steuern der Heizvorrichtung mittels des Heizvorrichtungssteuersignals und/oder ein Steuern des Einlassventils mittels eines/des Ventilsteuersignals umfassen.

Unter anderem sei auf die obigen Ausführungen verwiesen. Das Steuern der Heizvorrichtung mittels des Heizvorrichtungssteuersignals kann umfassen, dass der Betrieb der Heizvorrichtung gemäß dem Heizvorrichtungssteuersignal gestartet, gestoppt oder angepasst wird. Das kann sofort oder mit einer durch das Heizvorrichtungssteuersignal vorgegebenen Verzögerung erfolgen. Das Steuern eines/des Einlassventils mittels eines/des Ventilsteuersignals kann umfassen, dass das Einlassventil gemäß dem Ventilsteuersignal geöffnet oder geschlossen wird. Das kann sofort oder mit einer durch das Ventilsteuersignal vorgegebenen Verzögerung erfolgen.

Gemäß der vorliegenden Offenbarung kann das Steuern der Heizvorrichtung umfassen, mit vorgegebener Verzögerung und/oder nach Erfüllen mindestens eines weiteren Kriteriums und/oder nach Benutzerfreigabe oder unmittelbar bei Erfüllen des mindestens einen Kriteriums die Heizvorrichtung gemäß dem Heizvorrichtungssteuersignal zu steuern, insbesondere das Heizen gemäß dem Heizvorrichtungssteuersignal zu starten oder zu stoppen oder anzupassen.

In diesem Zusammenhang sei auch auf die Ausführungen oben verwiesen. Insbesondere kann die Verzögerung durch eine durch das Heizvorrichtungssteuersignal vorgegebene Verzögerung erreicht werden.

Insbesondere kann, wenn das Steuern der Heizvorrichtung nach Erfüllen mindestens eines weiteren Kriteriums erfolgt, das Heizvorrichtungssteuersignal sofort nach Erfüllen des weiteren Kriteriums erfolgen.

Beispielsweise ist denkbar, dass zwei druckbezogene Kriterien oder ein druckbezogenes Kriterium und ein nicht-druckbezogenes Kriterium erfüllt sein müssen, damit das Heizvorrichtungssteuersignal erzeugt wird.

Das Steuern nach Benutzerfreigabe kann beispielsweise umfassen, dass, sobald erkannt wird, dass das Kriterium erfüllt ist, eine Aufforderung an einen Benutzer ausgegeben wird, zu bestätigen, das Heizvorrichtungssteuersignal zu erzeugen und/oder die Heizvorrichtung entsprechend zu steuern.

Gemäß der vorliegenden Offenbarung kann das Steuern des Einlassventils umfassen, mit vorgegebener Verzögerung und/oder nach Erfüllen mindestens eines weiteren Kriteriums und/oder nach Benutzerfreigabe oder unmittelbar bei Erfüllen des mindestens einen Kriteriums das Einlassventil gemäß dem Ventilsteuersignal zu steuern, insbesondere das Ventil gemäß dem Ventilsteuersignal zu öffnen oder zu schließen.

In diesem Zusammenhang sei auch auf die Ausführungen oben verwiesen. Insbesondere kann die Verzögerung durch eine durch das Ventilsteuersignalsignal vorgegebene Verzögerung erreicht werden. Insbesondere kann, wenn das Steuern des Einlassventils nach Erfüllen mindestens eines weiteren Kriteriums erfolgt, das Ventilsteuersignal sofort nach Erfüllen des weiteren Kriteriums erfolgen.

Beispielsweise ist denkbar, dass zwei druckbezogene Kriterien oder ein druckbezogenes Kriterium und ein nicht-druckbezogenes Kriterium erfüllt sein müssen, damit das Ventilsteuersignal erzeugt wird.

Das Steuern nach Benutzerfreigabe kann beispielsweise umfassen, dass, sobald erkannt wird, dass das Kriterium erfüllt ist, eine Aufforderung an einen Benutzer ausgegeben wird, zu bestätigen, das Ventilsteuersignal zu erzeugen und/oder das Einlassventil entsprechend zu steuern.

Gemäß der vorliegenden Offenbarung kann das Verfahren einen Prüfprozess zum Prüfen eines Niveausensors in einem Flüssigkeitsbehälter des Dialysesystems, insbesondere einem dem Entgasungsabschnitt in Flussrichtung vorgelagerten Behälter, umfassen, wobei der überwachte Druck und/oder die Steuerdaten als erste Eingangsdaten für den Prüfprozess verwendet werden, wobei Messdaten des Niveausensors als zweite Eingangsdaten für den Prüfprozess verwendet werden, wobei der Prüfprozess ausgibt, dass der Niveausensor ordnungsgemäß funktioniert,

wenn sowohl die Messdaten des Niveausensors als auch der Druck und/oder die Steuerdaten ein Unterschreiten einer vorgegebenen Mindestmenge von Flüssigkeit im Dialysesystem anzeigen, oder wenn sowohl die Messdaten des Niveausensors als auch der Druck und/oder die Steuerdaten ein Vorhandensein einer vorgegebenen Mindestmenge von Flüssigkeit im Dialysesystem anzeigen,

wobei der Prüfprozess andernfalls ausgibt, dass der Niveausensor nicht ordnungsgemäß funktioniert.

Mit anderen Worten wird der Prüfprozess ermöglichen eine Übereinstimmung der verschiedenen Bestimmungsmethoden zu prüfen. Wenn keine Übereinstimmung vorliegt, ist von einem nicht funktionierendem Niveausensor auszugehen. Den Drucksensor als Referenz zu verwenden, ist vorteilhaft, weil eine Fehlfunktion des Drucksensors zuverlässiger erkennbar ist als eine Fehlfunktion eines Niveausensors.

Gemäß der vorliegenden Offenbarung kann, insbesondere immer oder zumindest in einem Fall, in dem der/ein Niveausensor und/oder der/ein Temperatursensor ausfällt, eine Steuerung des Heizens und/oder eines/des Einlassventils teilweise oder vollständig gemäß dem überwachten Druck erfolgen.

Im Normalbetrieb kann beispielsweise das Einlassventil gemäß der Messung eines/des Niveausensors im Heizbehälter gesteuert werden und/oder die Heizvorrichtung gemäß einer Temperaturmessung eines/des Temperatursensors. Da das Flüssigkeitsniveau bzw. die Temperatur unmittelbar und den Zulauf von Flüssigkeit bzw. an den Heizbetrieb der Heizvorrichtung gekoppelt sind, ist dies eine einfache Variante. Allerdings ist denkbar, zumindest in einem Notbetrieb, also wenn das Steuern basierend auf gemessenen Füllstandswerten bzw. Temperaturen nicht möglich ist, und optional auch im Normalbetrieb, den überwachten Druck für die Steuerung zu berücksichtigen, insbesondere, die Steuerung ohne die Verwendung von gemessenen Füllstandswerten bzw. Temperaturen durchzuführen.

So kann eine höhere Zuverlässigkeit erreicht werden.

Gemäß der vorliegenden Offenbarung kann nur das Starten und optional auch das Stoppen des Heizens druckabhängig gemäß dem überwachten Druck erfolgen und das Anpassen des Heizens im laufenden Heizbetrieb druckunabhängig erfolgen. Das gilt insbesondere für einen Normalbetrieb. Im Normalbetrieb kann beispielsweise im laufenden Heizbetrieb eine temperaturbasierte Steuerung, insbesondere Anpassung der Heizvorrichtung erfolgen, wobei die Temperatur beispielsweise im Heizbehälter gemessen werden kann.

Die Kombination ermöglicht, eine einfache und unmittelbare Anpassung der Heizleistung, um die gewünschte Temperatur bereitzustellen, und eine Möglichkeit, das Starten und ggf. Stoppen des Heizens unabhängig von der Temperatur zu machen, die für diese beiden Vorgänge nicht immer ein zuverlässiges Kriterium ist.

Optional kann, wie oben erläutert, für einen Notbetrieb, beispielsweise wenn eine temperaturbasierte Anpassung bzw. Steuerung des Heizens im laufenden Heizbetrieb nicht möglich ist, auch im laufenden Heizbetrieb eine druckabhängige Steuerung bzw. Anpassung der Heizvorrichtung erfolgen, insbesondere beispielsweise ein Herunterregeln der Heizleistung.

Die vorliegende Offenbarung stellt auch ein System zur Datenverarbeitung bereit, wobei das System zum Durchführen des Verfahrens gemäß der vorliegenden Offenbarung, insbesondere wie oben beschrieben, ausgebildet ist.

Die vorliegende Offenbarung stellt auch ein Dialysesystem bereit, das das System zur Datenverarbeitung umfasst und ferner ein Fluidsystem umfassend einen Entgasungsabschnitt zum Entgasen von Flüssigkeit, einen Sensor zum Bestimmen des Drucks in dem Entgasungsabschnitt und eine Heizvorrichtung zum Heizen von im Dialysesystem befindlicher Flüssigkeit umfasst. Das Dialysesystem kann optional ein Einlassventil und/oder einen Flüssigkeitsbehälter und/oder einen Niveausensor umfassen.

Insbesondere kann die Heizvorrichten zum Heizen von Flüssigkeit in einem Heizbehälter des Dialysesystems ausgebildet sein, insbesondere einen Heizstab oder ein Heizrohr umfassen.

Der Drucksensor bzw. die Drucksensoren können in einem oder mehreren Bereichen des Entgasungsabschnitts angeordnet sein.

Der Entgasungsabschnitt kann eine Entgasungskammer, eine Pumpe und eine Drossel umfassen. Der Druck kann beispielsweise in Flussrichtung zwischen der Drossel und der Pumpe gemessen werden. Die Entgasungskammer kann in Flussrichtung zwischen dem Bereich der Druckmessung und der Pumpe angeordnet sein.

Das Überwachen des Drucks kann umfassen, dass mindestens ein Drucksensor im Entgasungsabschnitt angeordnet ist und, z.B. in regelmäßigen Abständen oder kontinuierlich, den Druck misst und Messwerte bereitstellt. Das Überwachen kann ein, beispielsweise regelmäßiges oder kontinuierliches, Erfassen von Messwerten eines oder mehrerer Drucksensoren umfassen.

Die Entgasungskammer kann so gestaltet sein, dass sie viele Ecken und Kanten aufweist, die ein Abscheiden der Luft begünstigen. Es kann sich um eine in diesem Bereich übliche Entgasungskammer handeln.

Die vorliegende Offenbarung stellt auch ein Computerprogrammprodukt bereit, umfassend Befehle, die bei der Ausführung des Programms durch einen Computer diesen veranlassen, das Verfahren gemäß der vorliegenden Offenbarung, insbesondere wie oben beschrieben, auszuführen.

Die vorliegende Offenbarung stellt auch ein computerlesbares Medium bereit, umfassend Befehle, die bei der Ausführung des Programms durch einen Computer diesen veranlassen, das Verfahren gemäß der vorliegenden Offenbarung, insbesondere wie oben beschrieben, auszuführen.

Die Merkmale und Vorteile, die im Zusammenhang mit dem Verfahren beschrieben wurden, gelten entsprechend auch für das System zur Datenverarbeitung, das Dialysesystem, das Computerprogrammprodukt und das computerlesbare Medium.

KURZE BESCHREIBUNG DER FIGUREN

Weitere Beispiele und Ausführungsformen werden im Folgenden anhand der Figuren erläutert. Dabei zeigen:

Figur 1     eine schematische Darstellung eines Verfahrens gemäß der vorliegenden Offenbarung;

Figur 2     eine schematische Darstellung eines Systems gemäß der vorliegenden Offenbarung;

Figur 3     eine schematische Darstellung eines Systems gemäß der vorliegenden Offenbarung;

Figur 4     eine schematische Darstellung eines Druckverlaufs in einem Entgasungsabschnitt;

Figur 5     eine schematische Darstellung eines Verlaufs der Standardabweichung des Drucks in einem Entgasungsabschnitt;

Figur 6     eine schematische Darstellung eines Verfahrens gemäß der vorliegenden Offenbarung.

DETAILLIERTE BESCHREIBUNG DER ERFINDUNG

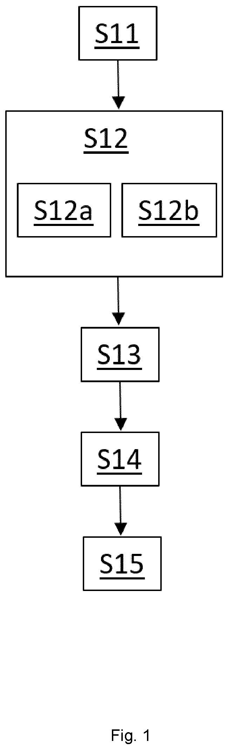

In Figur 1 ist schematisch ein computer-implementiertes Verfahren für ein Dialysesystem Erzeugen von Steuerdaten zum druckbasierten Steuern des Dialysesystems gemäß der vorliegenden Offenbarung dargestellt.

Das Verfahren umfasst, in Schritt S11, ein Überwachen des Drucks in einem Entgasungsabschnitt eines Fluidsystem des Dialysesystems.

Das Verfahren umfasst, in Schritt S12, ein Erzeugen von Steuerdaten, wenn erkannt wird, dass der Druck mindestens ein Kriterium erfüllt. Das Erzeugen der Steuerdaten umfasst ein Erzeugen 12a eines Heizvorrichtungssteuersignals $S_H$ für eine Heizvorrichtung des Dialysesystems.

Optional kann das Erzeugen der Steuerdaten ferner ein Erzeugen S12b eines Ventilsteuersignals $S_V$ für ein Einlassventil des Dialysesystems umfasst, welches die Flüssigkeitszufuhr in das Fluidsystem des Dialysesystems steuert, wobei das mindestens eine Kriterium ein Öffenkriterium umfasst, wobei, wenn der Druck das Öffenkriterium erfüllt, ein Signal zum Öffnen des Einlassventils (Öffensignal) erzeugt wird, und/oder wobei das mindestens eine Kriterium ein Schließkriterium umfasst, wobei, wenn der Druck das Schließkriterium erfüllt, ein Signal zum Schließen (Schließsignal)des Einlassventils erzeugt wird.

Das Verfahren kann optional ein Steuern S13 der Heizvorrichtung mittels des Heizvorrichtungssteuersignals und/oder ein Steuern S14 des Einlassventils mittels des Ventilsteuersignals umfassen.

Das optionale Steuern S13 der Heizvorrichtung kann umfassen, mit vorgegebener Verzögerung und/oder nach Erfüllen mindestens eines weiteren Kriteriums und/oder nach Benutzerfreigabe oder unmittelbar bei Erfüllen des mindestens einen Kriteriums die Heizvorrichtung gemäß dem Heizvorrichtungssteuersignal zu steuern, insbesondere das Heizen gemäß dem Heizvorrichtungssteuersignal zu starten oder zu stoppen oder anzupassen. Das jeweilige Steuersignal kann derart sein, dass die jeweilige durch das Steuersignal vorgegebene Aktion verzögert ausgeführt wird, insbesondere mit einer durch das Steuersignal vorgegebenen Verzögerung.

Das optionale Steuern S14 des Einlassventils kann umfassen, mit vorgegebener Verzögerung und/oder nach Erfüllen mindestens eines weiteren Kriteriums und/oder nach Benutzerfreigabe oder unmittelbar bei Erfüllen des mindestens einen Kriteriums das Einlassventil gemäß dem Ventilsteuersignal zu steuern, insbesondere das Ventil gemäß dem Ventilsteuersignal zu öffnen oder zu schließen. Das jeweilige Steuersignal kann derart sein, dass die jeweilige durch das Steuersignal vorgegebene Aktion verzögert ausgeführt wird, insbesondere mit einer durch das Steuersignal vorgegebenen Verzögerung.

Das Verfahren kann optional einen Prüfprozess S15 zum Prüfen eines Niveausensors in einem Flüssigkeitsbehälter des Dialysesystems umfassen, der beispielsweise in Flussrichtung dem Entgasungsabschnitt vorgelagert sein kann. Insbesondere kann der Flüssigkeitsbehälter so angeordnet sein, dass Flüssigkeit aus dem Flüssigkeitsbehälter unmittelbar in den Entgasungsabschnitt gelangen kann. Der überwachte Druck und/oder die Steuerdaten können als erste Eingangsdaten und die Messdaten des Niveausensors als zweite Eingangsdaten für den Prüfprozess verwendet werden.

Beispielhaft kann der Prüfprozess ausgeben, dass der Niveausensor ordnungsgemäß funktioniert, wenn sowohl die Messdaten des Niveausensors als auch der Druck und/oder die Steuerdaten ein Unterschreiten einer vorgegebenen Mindestmenge von Flüssigkeit im Dialysesystem anzeigen, oder wenn sowohl die Messdaten des Niveausensors als auch der Druck und/oder die Steuerdaten ein Vorhandensein einer vorgegebenen Mindestmenge von Flüssigkeit im Dialysesystem anzeigen. Der Prüfprozess kann andernfalls ausgeben, dass der Niveausensor nicht ordnungsgemäß funktioniert.

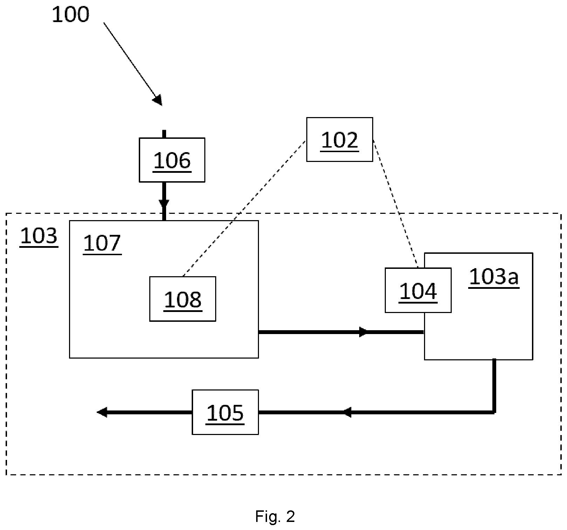

In Figur 2 ist beispielhaft ein System 102 zur Datenverarbeitung gemäß der vorliegenden Offenbarung schematisch dargestellt, wobei das System 102 zum Durchführen des Verfahrens gemäß der vorliegenden Offenbarung, beispielsweise wie im Zusammenhang mit Figur 1 beschrieben, ausgebildet ist. Das System kann ein oder mehrere Recheneinheiten umfassen, auf denen das Verfahren zentral oder dezentral durchgeführt und/oder gesteuert wird.

Wie in der Figur 2 gezeigt, kann das System 102 Teil eines, hier schematisch dargestellten, Dialysesystems 100 gemäß der vorliegenden Offenbarung sein. Das Dialysesystem kann ferner ein Fluidsystem 103 umfassend einen Entgasungsabschnitt 103a zum Entgasen von Flüssigkeit, einen Sensor 104 zum Bestimmen des Drucks in dem Entgasungsabschnitt 103a und eine Heizvorrichtung 105 zum Heizen von im Dialysesystem 100 befindlicher Flüssigkeit umfassen. Optional kann das System ein Einlassventil 106 und/oder einen Flüssigkeitsbehälter 107 und/oder einen Niveausensor 108 im Flüssigkeitsbehälter 107 umfassen, wobei der Flüssigkeitsbehälter Teil des Fluidsystems ist.

Weitere Merkmale und Vorteile von Verfahren und Systemen gemäß der vorliegenden Erfindung sind im Folgenden erläutert.

Die vorliegende Offenbarung zeigt Systeme und Verfahren zum Ein- und Ausschalten eines Heizvorgangs, wobei der Vorgang in Abhängigkeit eines gemessenen Drucks gesteuert/geregelt wird.

Die vorliegende Offenbarung beschreibt ein Verfahren für ein Dialysesystem, also System zum Sammeln, Entgasen und Erwärmen von Flüssigkeit im Rahmen einer extrakorporalen Blutbehandlung. Bei einer solchen Behandlung kann Wasser kontinuierlich entgast und erwärmt werden und anschließend mit einer basischen und einer sauren Komponente zu der sogenannten Dialysierflüssigkeit gemischt werden, welche dann einem Blutfilter bzw. Dialysator zugeführt wird, um dort mit Blut in diffusiven Austausch zu treten.

Gegenstand der Offenbarung ist unter anderem das Bereitstellen von Steuersignalen zum Steuern von Komponenten eines Dialysesystems, insbesondere der Heizvorrichtung und optional einem Ventil, auf Basis einer Druckmessung, die eine indirekte Füllstandsmessung für einen dem Entgasungsabschnitt in Flussrichtung vorgelagerten Flüssigkeitsbehälter darstellen kann.

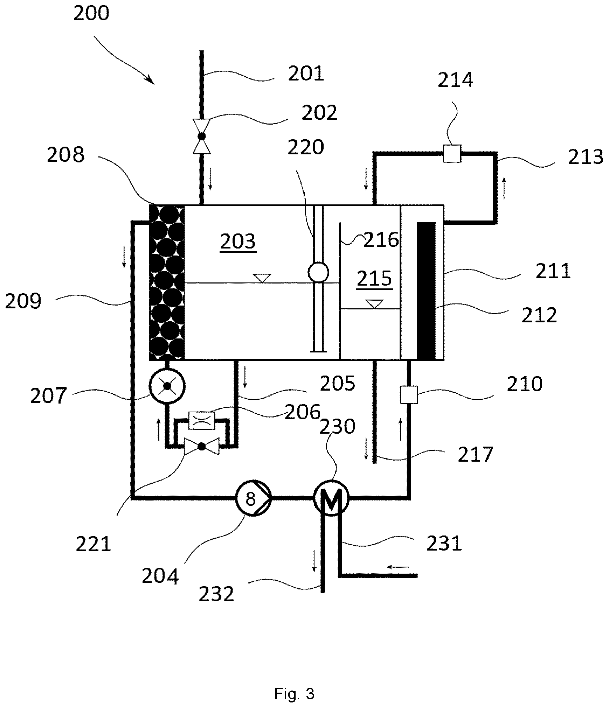

Figur 3 zeigt schematisch ein beispielhaftes System 200 (Dialysesystem) zum Sammeln, Entgasen und Erwärmen von Flüssigkeit, hier beispielsweise Wasser. Über den Wasserzulauf 201 strömt Wasser bei geöffnetem Ventil 202 in die Vorlaufkammer 203, auch als Kammer 203 bezeichnet. Angetrieben von der Pumpe 204 strömt das Wasser aus der Kammer 203 durch die Leitung 205 und die Druckmessvorrichtung 207 (unten auch als Sensor bezeichnet) in die Entgasungskammer 208. Stromauf der Druckmessvorrichtung 207 befindet sich eine Drossel 206 zum Verringern des Querschnitts der Leitung 205. Bei Bedarf kann das Wasser auch durch das Ventil 221 geleitet werden. Stromab der Pumpe 204 durchströmt das Wasser einen Rekuperator 230, in welchem es die Wärme des zulaufenden Fluids in Leitung 231 aufnehmen kann. Die Vorlaufkammer 203 kann beispielsweise eine Öffnung am oberen Rand bzw. im oberen Deckel der Vorlaufkammer aufweisen. Durch diese Öffnung kann Luft entweichen, die bei der Entgasung ausgeschieden wird.

Das wärmeabgebende Fluid verlässt den Rekuperator 230 durch die Leitung 232. Das erwärmte Wasser strömt anschließend weiter durch einen optionalen Temperatursensor 210 in eine Temperierkammer 211, wo es von einer Heizvorrichtung 212 weiter erwärmt wird. Das Wasser verlässt die Temperierkammer 211 über die Leitung 213 und gelangt in die

Entnahmekammer 215, die von der Vorlaufkammer 203 durch eine Trennwand 216 getrennt ist. In oder an der Leitung 213 befindet sich eine Temperaturmessvorrichtung 214. Übersteigt der Füllstand in der Kammer 215 die Höhe der Trennwand 216, so kann das Wasser in die Kammer 203 überlaufen. Aus der Entnahmekammer 215 kann das temperierte Wasser durch die Leitung 217 entnommen werden.

Figur 3 zeigt auch eine Leitung 222, die in die Kammer 203 mündet und beispielsweise für eine Zirkulation während einer Desinfektionsphase vorgesehen ist.

Im Bereich der Leitung 213 kann optional eine hier nicht gezeigte Abzweigung vorgesehen sein, durch die Wasser entnommen werden kann. Beispielsweise kann eine andas Fluidsystem angeschlossene, mit Natriumhydrogencarbonat gefüllte Kartusche mit so entnommenem Wasser befüllt werden. Das gelöste Salz kann im weiteren Verlauf zur Herstellung der Dialysierflüssigkeit verwendet werden. Neben der Leitung 217 kann zusätzlich also auch eine weitere Entnahmeleitung existieren.

Der Füllstand in Kammer 203 kann durch den optionalen Niveausensor 220 bestimmt werden. Wird die Kammer 203 z.B. als voll erkannt, schließt das Ventil 202. Es öffnet wieder, sobald die Kammer 203 entleert wird bzw. als leer oder fast leer erkannt wurde. Dies kann zeitgesteuert oder durch Betrachtung weiterer Pegelstände erfolgen. Auch eine Leitung 209 ist gezeigt.

Die vorliegende Offenbarung sieht vor, den Heizvorgang durch die Heizvorrichtung 212 nur dann durchzuführen, wenn bei laufender Pumpe 204 ein Unterduck an der Druckmessvorrichtung 207 gemessen wird.

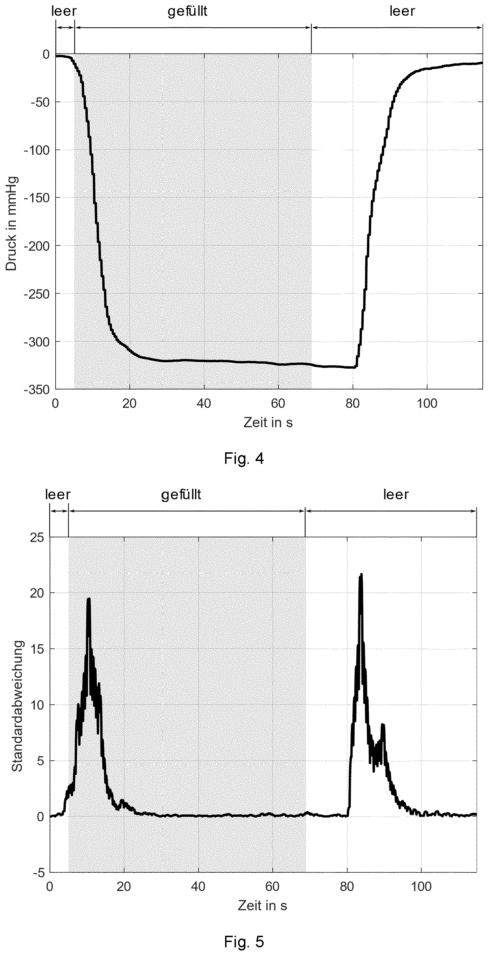

Figur 4 zeigt beispielhaft für eine Umdrehungsrate von 1500 rpm der Pumpe 204, wie der durch Sensor 207 gemessene Druck bei etwa 0 mmHg bzw. 0 kPa liegt, wenn die Vorlaufkammer 203 leer bzw. nahezu leer ist. Der Druck pendelt sich bei gefüllter Kammer 203 auf einen konstanten unteren Druckwert ein, der von der Drehzahl bzw. Förderrate der Pumpe 204 abhängig ist. Bedingt durch das Restvolumen in den Kammern und Leitungen steigt der Druck zeitverzögert wieder auf einen Wert von ca. 0 mmHg bzw. 0 kPa, wenn die Kammer 203 geleert wurde. Der Druckabfall (in diesem Beispiel bei ca. 5 s) kann genutzt werden, um ein Einschalten der Heizvorrichtung 212 zu bewirken. Der Druckanstieg (in diesem Beispiel bei ca. 80 s) kann genutzt werden, um ein Ausschalten der Heizvorrichtung 212 zu bewirken.

Beispielsweise kann ein Schwellwert herangezogen werden. Ist die Kammer 203 initial leer, d.h. der Druck etwa 0 mmHg bzw. 0 kPa, kann der Heizvorgang gestartet werden, sobald der Druck bspw. einen Wert von -20 mmHg oder -2,7 kPa unterschreitet.

Wird die Kammer 203 dagegen geleert, kann der Heizvorgang gestoppt werden, sobald der Druck den konstanten Druckwert im gefüllten Zustand (hier ca. -320 mmHg oder -42,7 kPa) um einen gewissen Wert (z.B. 20 mmHg oder 2,7 kPa) überschreitet.

Alternativ kann die gleitende Standardabweichung des Drucks ausgewertet werden. Diese ist beispielhaft in Figur 5 dargestellt. Abhängig davon, welcher Zustand zuletzt vorlag, d.h., ob geheizt wurde oder nicht, kann beim Überschreiten eines Schwellwerts der Standardabweichung (z.B. der Wert 5 bei den Angaben in mmHg) der Heizvorgang gestartet oder gestoppt werden. Es versteht sich, dass der Wert abhängig von der gewählten Druckeinheit ist. Wird der Druck in kPa angegeben, ist der Schwellwert der Standardabweichung natürlich entsprechend höher.

Mindestens eine der Leitungen 201, 205, 209, 213, 217, 231 und 232 kann als Schlauch oder Rohr oder sonstige Verbindung ausgelegt sein.

Gängige Niveausensoren sind als Schwimmer ausgelegt, die sich z.B. entlang eines Stabes im Behälter mit dem Füllstand bewegen. Dabei kann es dazu kommen, dass der Schwimmer klemmt und sich nicht weiter bewegen lässt. Verbleibt der Schwimmer bspw. in oberer Position, obwohl der Füllstand sinkt, kann das nicht registriert werden, sodass das Einlaufventil geschlossen bleibt und die Kammern leer laufen. Verbleibt der Schwimmer dagegen bspw. in unterer Position, kann es zu einem Überlaufen der Kammern kommen, da das Einlaufventil dauerhaft geöffnet bleibt.

Durch die gleichzeitige Betrachtung des Drucks am Sensor 207 kann ein Klemmen des Schwimmers am Niveausensor 220 erkannt werden.

Klemmt der Schwimmer bspw. in oberer Position, kann die Kammer 203 leerlaufen, was bei laufender Pumpe 204 durch einen Druckanstieg an der Messvorrichtung 207 bemerkbar wird.

Auch ein Klemmen in unterer Position kann erkannt werden, wenn z.B. zusätzlich die Temperatur in der Leitung 217 betrachtet wird (Messvorrichtung in FIG. 3 nicht dargestellt). Ist das Ventil 202 geöffnet, läuft das Wasser in Kammer 203 nach einer gewissen Zeit über die Trennwand 216 in die Entnahmekammer 215. Da das Wasser in Kammer 203 kälter ist als das bereits erwärmte Wasser in Kammer 215, wäre ein Temperaturabfall in der Leitung 217 zu erkennen bzw. ist die Temperatur dort geringer als an der Messvorrichtung 214.

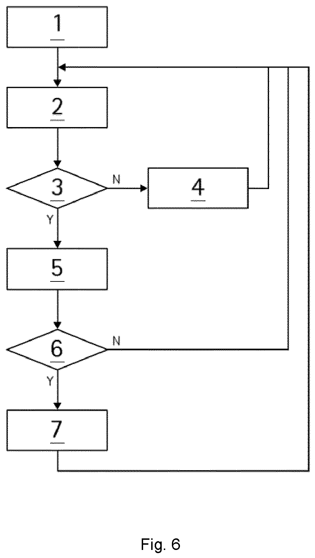

Ein weiteres Beispiel der vorliegenden Offenbarung schlägt vereinfacht dargestellt folgende Verfahrensschritte für das Starten eines Heizvorgangs vor, wenn in einer Kammer des Vorlaufbehälters, beispielsweise in einem dem Entgasungsabschnitt unmittelbar vorgelagerten Flüssigkeitsbehälter, kein gewöhnlicher Niveausensor enthalten ist. Diese Schritte sind in Figur 6 als Flussdiagramm gezeigt.

1. Starten Pumpe 204, sodass Wasser (falls vorhanden) aus Kammer 203 angesaugt werden kann. Das Bypassventil 221 ist dabei geschlossen, sodass das Wasser durch die Drossel 206 fließen müsste, was zum Aufbau eines Unterdrucks führen würde.

2. Messen des Druckes an der Vorrichtung 207.

3. Vergleichen des gemessenen Druckes mit einem Schwellwert. Im einfachsten Fall liegt dieser Schwellwert bei 0 mmHg bzw. 0 kPa und insbesondere zwischen 0 mmHg bzw. 0 kPa und -50 mmHg oder -6,7 kPa. Liegt der gemessene Wert oberhalb des Schwellwerts, ist der Vorlaufbehälter leer ($\rightarrow$ gehe zu Schritt 4). Liegt der gemessene Wert unterhalb des Schwellwerts, wurde Wasser gefördert, was darauf hindeutet, dass der Vorlaufbehälter gefüllt ist ($\rightarrow$ gehe zu Schritt 5).

4. Öffnen des Ventils 202, für den Fall, dass der gemessene Wert oberhalb des Schwellwerts lag. Das Öffnen kann alternativ nur für eine vorbestimmte Zeit erfolgen. Nach Ablauf dieser Zeit, kann das Ventil 202 automatisch wieder schließen, ohne auf weitere Bedingungen warten zu müssen. Schritt 5 würde in diesem Fall entfallen.

5. Schließen des Ventils 202, falls es nicht bereits geschlossen ist (vgl. Schritt 4).

6. Optional kann vor Schritt 7 noch abgefragt werden, ob ein Erwärmen programmseitig oder nutzerseitig gewollt ist (grundsätzlich ist der Vorlaufbehälter gefüllt und die Grundvoraussetzung zum Erwärmen des Wassers erfüllt).

7. Der Heizvorgang mittels Heizvorrichtung 212 wird eingeleitet und durchgeführt.

Schritte 2 bis 7 können wiederholt werden.

Für das Bestimmen der vorbestimmten Zeit in Punkt 4 können beispielhaft folgende Überlegungen herangezogen werden.

Die Einstellung des Druckminderventils am Wassereingang ist in gewissem Maße bekannt. Beispielsweise ist häufig die Einstellung des Druckminderventils herstellerseitig voreingestellt mit einer angegebenen Toleranz. Somit ist die Menge an einströmendem Wasser $\dot{V}_{zu}$ bei geöffnetem Wasserzulauf 202 (bei konstantem Leitungsdruck) abschätzbar, ggf. innerhalb der angegebenen Toleranz. Der Zustrom über den Wassereingang liegt beispielsweise zwischen 30 und 40 ml/s.

Das aufzufüllende Volumen $\Delta V$ der Kammer 203 mit dem Wassereinlauf kann beispielsweise ca. 300 ml betragen (Boden der Kammer bis oberer Anschlagpunkt des Schwimmerschalters). Mit einem Zufluss von 30 ml/s ist die Kammer innerhalb von 10 s vollständig gefüllt. Bei einem Zufluss $\dot{V}_{zu}$ von 40 ml/s ist die Kammer in 7,5 s gefüllt. Diese Werte sind ohne durch die Leitung 217 abfließenden Volumenstrom $\dot{V}_{ab}$ berechnet.

Für die Auslegung der Öffnungsdauer des Wasserzulaufventils 202 kann eine flussabhängige Parametrierung sinnvoll sein.

Die Menge an ausströmendem Wasser $\dot{V}_{ab}$ an Position 217 (aus Kammer 215 mittels einer nicht gezeigten Pumpe) kann durch Flussbilanzierung durch Bilanzkammern (ebenfalls nicht gezeigt) bestimmt werden. Flussbilanzierung mit Bilanzkammern kann mit bekannten Methoden erfolgen. Über einen Steg 216 ist ein Überlauf von Kammer 215 in Kammer 203 möglich. Das Volumen von übertretendem Wasser wird vom Volumenstrom $\dot{V}_{EP}$ der Fördereinrichtung 204 erzeugt. Der Volumenstrom $\dot{V}_{EP}$ der Fördereinrichtung 204 liegt baulich bedingt in der Regel etwas höher als der Volumenstrom der (nicht gezeigten) Dialysierflüssigkeitspumpe $\dot{V}_{ab}$. Das abfließende Wasser aus Kammer 215 reduziert das über den Steg 216 tretende Volumen durch die Fördereinrichtung 204 (Pumpe) um den bilanzierten Fluss.

Die flussabhängige Öffnungsdauer kann somit folgendermaßen dynamisch bestimmt werden:

$$t = \frac{\Delta V}{\dot{V}_{zu} - \dot{V}_{ab}}$$

Der therapietypische Dialysierflüssigkeitsfluss liegt zwischen 300 ml/min (5 ml/s) und 800 ml/min (13,33 ml/s). Die Öffnungsdauer t wird nach der beschriebenen Formel umso größer, je größer der Ablaufstrom, generiert durch die Dialysierflüssigkeitspumpe, ist.

Des Weiteren ist die Abhängigkeit vom Zirkulationsventil zu betrachten (z.B. bei Desinfektion). Beim Spülen über den Zirkulationskreis läuft das geförderte Volumen $\dot{V}_{ab}$ direkt von den Bilanzkammern bzw. der Spülbrücke kommend zurück in Kammer 203. Dabei fließt normalerweise keine Flüssigkeit in den Abfluss, so dass der Füllstand nicht absinkt. Der Volumenstrom Vab wird dadurch zu Null. Die beschriebene Formel behält dabei ihre Gültigkeit. Die Zulaufleitung für die Zirkulation während der Desinfektionsphase ist in Figur 3 mit dem Bezugszeichen 222 bezeichnet. In dem in der Figur gezeigten Beispiel strömt, wenn das Zirkulationsventil geöffnet ist, die Flüssigkeit durch Leitung 222 zurück in Kammer 203. Die Leitung 231, die zum Wärmetauscher 230 führt, und die Leitung 232, die mit dem Abfluss verbunden ist, werden dann in diesem Beispiel nicht mehr durchströmt.

Obwohl die Erfindung in den Zeichnungen und der vorstehenden Beschreibung detailliert dargestellt und beschrieben ist, sind diese Darstellungen und Beschreibungen als beispielhaft und nicht einschränkend zu betrachten. Die Erfindung ist nicht auf die offengelegten Ausführungsformen beschränkt. In Anbetracht der vorstehenden Beschreibung und der Zeichnungen wird es für den Fachmann offensichtlich sein, dass im Rahmen der Erfindung, wie sie in den Ansprüchen definiert ist, verschiedene Modifikationen vorgenommen werden können.

**Patentansprüche**

1. Computer-implementiertes Verfahren für ein Dialysesystem zum Erzeugen von Steuerdaten zum druckbasierten Steuern des Dialysesystems, wobei das Verfahren umfasst:

   Überwachen (S11) des Drucks in einem Entgasungsabschnitt eines Fluidsystem des Dialysesystems,
   Erzeugen (S12) von Steuerdaten, wenn erkannt wird, dass der Druck mindestens ein Kriterium erfüllt,
   wobei das Erzeugen der Steuerdaten ein Erzeugen (S12a) eines Heizvorrichtungssteuersignals für eine Heizvorrichtung des Dialysesystems umfasst.

2. Verfahren nach Anspruch 1,

   wobei das mindestens eine Kriterium ein erstes Kriterium, das sich auf einen Druckwert bezieht, umfasst, und/oder
   wobei das mindestens eine Kriterium ein zweites Kriterium, das sich auf eine Druckänderung, insbesondere einen Druckanstieg und/oder einen Druckabfall, bezieht, umfasst.

3. Verfahren nach einem der vorangegangenen Ansprüche,

   wobei das mindestens eine Kriterium ein Kriterium umfasst, dass ein tatsächlicher Druckwert einem Solldruckwert entspricht, und/oder
   wobei das mindestens eine Kriterium ein Kriterium umfasst, dass ein tatsächlicher Druckwert unter einen vorgegebenen Druckwert fällt oder dass ein tatsächlicher Druckwert über einen vorgegebenen Druckwert steigt oder dass ein tatsächlicher Druckwert sich mindestens um einen vorgegebenen Betrag ändert, insbesondere mindestens um den vorgegebenen Betrag steigt oder fällt, und/oder
   wobei das mindestens eine Kriterium ein Kriterium umfasst, dass eine gleitende Standardabweichung des Drucks über einen vorgegebenen Wert steigt.

4. Verfahren nach einem der vorangegangenen Ansprüche,

   wobei das mindestens eine Kriterium ein Startkriterium für das Starten der Heizvorrichtung umfasst, wobei, wenn der Druck das Startkriterium erfüllt, ein Startsignal als Heizvorrichtungssteuersignal erzeugt wird, und/oder wobei das mindestens eine Kriterium ein Stoppkriterium für das Stoppen der Heizvorrichtung umfasst, wobei, wenn der Druck das Stoppkriterium erfüllt, ein Stoppsignal als Heizvorrichtungssteuersignal erzeugt wird, und/oder wobei das mindestens eine Kriterium ein Anpassungskriterium für das Anpassen des Betriebs der Heizvorrichtung umfasst, wobei, wenn der Druck das Anpassungskriterium erfüllt, ein Anpassungssignal als Heizvorrichtungssteuersignal erzeugt wird.

5. Verfahren nach einem der vorangegangenen Ansprüche, wobei das mindestens eine Kriterium das Startkriterium umfasst und das Startkriterium umfasst, dass ein tatsächlicher Druckwert unter einen vorgegebenen Druckwert fällt oder dass ein tatsächlicher Druckwert mindestens um einen vorgegebenen Betrag fällt oder dass eine gleitende Standardabweichung des Drucks über einen vorgegebenen Wert steigt.

6. Verfahren nach einem der vorangegangenen Ansprüche,

wobei das Erzeugen der Steuerdaten ferner ein Erzeugen (S12b) eines Ventilsteuersignals für ein Einlassventil des Dialysesystems umfasst, welches die Flüssigkeitszufuhr in das Fluidsystem des Dialysesystems steuert, wobei das mindestens eine Kriterium ein Öffenkriterium umfasst, wobei, wenn der Druck das Öffenkriterium erfüllt, ein Signal zum Öffnen des Einlassventils erzeugt wird, und/oder wobei das mindestens eine Kriterium ein Schließkriterium umfasst, wobei, wenn der Druck das Schließkriterium erfüllt, ein Signal zum Schließen des Einlassventils erzeugt wird.

7. Verfahren nach einem der vorangegangenen Ansprüche, umfassend Steuern (S13) der Heizvorrichtung mittels des Heizvorrichtungssteuersignals und/oder Steuern (S14) eines/des Einlassventils mittels eines/des Ventilsteuersignals.

8. Verfahren nach Anspruch 7,

wobei das Steuern (S13) der Heizvorrichtung umfasst, mit vorgegebener Verzögerung und/oder nach Erfüllen mindestens eines weiteren Kriteriums und/oder nach Benutzerfreigabe oder unmittelbar bei Erfüllen des mindestens einen Kriteriums die Heizvorrichtung gemäß dem Heizvorrichtungssteuersignal zu steuern, insbesondere das Heizen gemäß dem Heizvorrichtungssteuersignal zu starten oder zu stoppen oder anzupassen, und/oder wobei das Steuern (S14) des Einlassventils umfasst, mit vorgegebener Verzögerung und/oder nach Erfüllen mindestens eines weiteren Kriteriums und/oder nach Benutzerfreigabe oder unmittelbar bei Erfüllen des mindestens einen Kriteriums das Einlassventil gemäß dem Ventilsteuersignal zu steuern, insbesondere das Ventil gemäß dem Ventilsteuersignal zu öffnen oder zu schließen.

9. Verfahren nach einem der vorangegangenen Ansprüche, umfassend einen Prüfprozess (S15) zum Prüfen eines Niveausensors in einem Flüssigkeitsbehälter des Dialysesystems,

wobei der Druck und/oder die Steuerdaten als erste Eingangsdaten für den Prüfprozess verwendet werden, wobei Messdaten des Niveausensors als zweite Eingangsdaten für den Prüfprozess verwendet werden, wobei der Prüfprozess ausgibt, dass der Niveausensor ordnungsgemäß funktioniert,

wenn sowohl die Messdaten des Niveausensors als auch der Druck und/oder die Steuerdaten ein Unterschreiten einer vorgegebenen Mindestmenge von Flüssigkeit im Dialysesystem anzeigen, oder wenn sowohl die Messdaten des Niveausensors als auch der Druck und/oder die Steuerdaten ein Vorhandensein einer vorgegebenen Mindestmenge von Flüssigkeit im Dialysesystem anzeigen,

wobei der Prüfprozess andernfalls ausgibt, dass der Niveausensor nicht ordnungsgemäß funktioniert.

10. Verfahren nach einem der vorangegangenen Ansprüche, wobei, insbesondere immer oder zumindest in einem Fall, in dem der/ein Niveausensor und/oder der/ein Temperatursensor ausfällt, eine Steuerung des Heizens und/oder eines/des Einlassventils teilweise oder vollständig gemäß dem Druck erfolgt.

11. Verfahren nach einem der vorangegangenen Ansprüche, wobei nur das Starten und optional das Stoppen des Heizens druckabhängig gemäß dem Druck erfolgt und das Anpassen des Heizens im laufenden Heizbetrieb druckunabhängig erfolgt.

12. System (102) zur Datenverarbeitung, wobei das System (102) zum Durchführen des Verfahrens nach einem der vorangegangenen Ansprüche ausgebildet ist.

13. Dialysesystem (100; 200) umfassend das System (102) zur Datenverarbeitung nach Anspruch 12, ferner umfassend

    ein Fluidsystem (103) umfassend einen Entgasungsabschnitt (103a; 205, 208, 209) zum Entgasen von Flüssigkeit,
einen Sensor (104; 207) zum Bestimmen des Drucks in dem Entgasungsabschnitt (3a),
eine Heizvorrichtung (105; 211, 212) zum Heizen von im Dialysesystem (;100) befindlicher Flüssigkeit,
optional ein Einlassventil (106; 202) und/oder einen Flüssigkeitsbehälter (107; 203) und/oder einen Niveausensor (108; 220).

14. Computerprogrammprodukt, umfassend Befehle, die bei der Ausführung des Programms durch einen Computer diesen veranlassen, das Verfahren nach einem der Ansprüche 1 bis 11 auszuführen.

15. Computerlesbares Medium, umfassend Befehle, die bei der Ausführung des Programms durch einen Computer diesen veranlassen, das Verfahren nach einem der Ansprüche 1 bis 11 auszuführen.

Fig. 1

Fig. 2

Fig. 3

Fig. 4

Fig. 5

Fig. 6

Europäisches Patentamt

European Patent Office

Office européen des brevets

## EUROPÄISCHER RECHERCHENBERICHT

**Nummer der Anmeldung**

EP 25 16 5308

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 142 234 B1 (GAMBRO LUNDIA AB [SE]) 23. Januar 2013 (2013-01-23) * Absatz [0026] - Absatz [0027]; Abbildung 1 * | 1-15 | INV. A61M1/16 |
| | ----- | | |
| X | US 2010/106071 A1 (WALLENBORG ANDERS [SE] ET AL) 29. April 2010 (2010-04-29) * Absatz [0042] - Absatz [0047]; Abbildung 2 * | 1-15 | |
| | ----- | | |
| A | US 2023/158220 A1 (FALLMAN OSKAR ERIK FRODE STYRBJORN [SE] ET AL) 25. Mai 2023 (2023-05-25) * Absatz [0100] * | 1-15 | |
| | ----- | | |

**RECHERCHIERTE SACHGEBIETE (IPC)**

A61M

Der vorliegende Recherchenbericht wurde für alle Patentansprüche erstellt

| Recherchenort | Abschlußdatum der Recherche | Prüfer |
|---|---|---|
| Den Haag | 18. August 2025 | Mata Vicente, Teresa |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 25 16 5308

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-08-2025

| Im Recherchenbericht angeführtes Patentdokument | | Datum der Veröffentlichung | Mitglied(er) der Patentfamilie | | | Datum der Veröffentlichung |
|---|---|---|---|---|---|---|
| EP 2142234 | B1 | 23-01-2013 | AU | 2007350967 | A1 | 23-10-2008 |
| | | | CA | 2683051 | A1 | 23-10-2008 |
| | | | CN | 101678161 | A | 24-03-2010 |
| | | | EP | 2142234 | A1 | 13-01-2010 |
| | | | ES | 2402306 | T3 | 30-04-2013 |
| | | | KR | 20100005210 | A | 14-01-2010 |
| | | | PL | 2142234 | T3 | 28-06-2013 |
| | | | US | 2012265117 | A1 | 18-10-2012 |
| | | | US | 2013319917 | A1 | 05-12-2013 |
| | | | WO | 2008125893 | A1 | 23-10-2008 |
| US 2010106071 | A1 | 29-04-2010 | AT | E524204 | T1 | 15-09-2011 |
| | | | CA | 2671010 | A1 | 05-06-2008 |
| | | | EP | 2101841 | A1 | 23-09-2009 |
| | | | ES | 2370985 | T3 | 26-12-2011 |
| | | | US | 2010106071 | A1 | 29-04-2010 |
| | | | WO | 2008065470 | A1 | 05-06-2008 |
| US 2023158220 | A1 | 25-05-2023 | AU | 2022396388 | A1 | 23-05-2024 |
| | | | CA | 3239001 | A1 | 01-06-2023 |
| | | | EP | 4436628 | A1 | 02-10-2024 |
| | | | JP | 2024541468 | A | 08-11-2024 |
| | | | US | 2023158220 | A1 | 25-05-2023 |
| | | | WO | 2023097147 | A1 | 01-06-2023 |

EPO FORM P0461

Für nähere Einzelheiten zu diesem Anhang : siehe Amtsblatt des Europäischen Patentamts, Nr.12/82